# EUROPÄISCHE PATENTSCHRIFT

(11) **EP 0 523 557 B1**
(45) Veröffentlichungstag und Bekanntmachung des Hinweises auf die Patenterteilung: **03.01.1996**
(21) Anmeldenummer: 92111743.8
(22) Anmeldetag: 10.07.1992
(51) Int. Cl.: C12Q 1/68

(54) **Immobilisierung von Nukleinsäuren**
Immobilisation of nucleic acids
Immobilisation des acides nucléiques

(30) Priorität: 16.07.1991 DE 4123540
(43) Veröffentlichungstag der Anmeldung: 20.01.1993
(73) Patentinhaber: BOEHRINGER MANNHEIM GMBH, D-68298 Mannheim (DE)
(72) Erfinder: Berner, Sibylle, Dipl.Chem.Dr., W-8900 Augsburg (DE); Köhler, Stephanie, Dipl.Biol.Dr., W-8130 Starnberg-Percha (DE); Kruse-Müller, Cornelia, Dipl.Chem.Dr., W-8132 Tutzing (DE); Seibl, Rudolf, Dr., W-8122 Penzberg (DE)

(56) Entgegenhaltungen:
- EP-A- 0 156 287
- EP-A- 0 225 807
- EP-A- 0 330 221
- EP-A- 0 357 336
- NUCLEIC ACIDS RESEARCH, Band 14, 1986, Arlington, VA (US); A.-C. SYVANEN et al., Seiten 5037-5048/
- NUCLEIC ACIDS RESEARCH, Band 18, Nr. 15, 1990, Arlington, VA (US); K. MISIURA et al., Seiten 4345-4354/
- NUCLEIC ACIDS RESEARCH, Band 18, Nr. 15, 1990, Arlington, VA (US); U. PIELES et al., Seiten 4355-4360/

## Beschreibung

Gegenstand der Erfindung sind ein Verfahren zum Nachweis von Nukleinsäuren, die Verwendung von speziellen Nukleinsäuren zur Immobilisierung von Nukleinsäuren und ein System zum Nachweis von Nukleinsäuren.

Die Immobilisierung von Nukleinsäuren ist auf vielen Gebieten wichtig, beispielsweise bei der Abtrennung von Nukleinsäuren von anderen Stoffen, bei der Isolierung von Nukleinsäuren einer speziellen Nukleotidsequenz aus einem Nukleinsäuregemisch heraus und außerdem beim Nachweis von Nukleinsäuren (Nukleinsäurediagnostik). Auf all diesen Gebieten werden die speziellen Affinitäten von Nukleinsäuren zu solchen Nukleinsäuren ausgenutzt, die eine dazu im wesentlichen komplementäre Nukleotidsequenz haben und unter bestimmten Bedingungen mit den gewünschten Nukleinsäuren hybridisieren können. Meist sind die dazu verwendeten komplementären Nukleinsäuren, die im folgenden als Fangprobe bezeichnet werden, an eine feste Phase, z.B. eine Membran, ein Gel, ein Kügelchen (bead) oder ein Gefäß (Tube) gebunden. In jüngerer Zeit wurde festgestellt, daß die Immobilisierung von Nukleinsäuren an feste Phasen über kovalent gebundene Fangprobes Nachteile in mehrerlei Hinsicht aufweist, z.B. ist die Immobilisierungsgeschwindigkeit herabgesetzt. Daher wurde vorgeschlagen, die Fangprobe über Affinitätsbindung spezifisch an die Festphase zu binden. Eine solche Bindung beispielsweise über Antikörper/Haptene oder Biotin/(Strept-)Avidin ermöglicht eine Hybridisierung der Nukleinsäure mit der Fangprobe in Flüssigkeit und anschließend Immobilisierung des gebildeten Hybrids. Eine solche Ausführungsform ist beispielsweise in der EP-A-0 139 489 beschrieben.

Aufgabe der vorliegenden Erfindung war es, die Effektivität der Immobilisierung zu erhöhen, um insbesondere sensitivere Tests auf Nukleinsäuren zu erhalten.

Gegenstand der Erfindung ist ein Verfahren zum Nachweis einer Analytnukleinsäure welches folgende Schritte enthält:
- Hybridisierung einer nachweisbar markierten Nukleinsäure oder eines nachweisbar markierten Nukleinsäurehybrids mit einer immobilisierbaren Nukleinsäuresonde;
- Immobilisierung des gebildeten Hybrids und
- Nachweis der Menge an immobilisiertem Hybrid über die Menge an Markierung,
   wobei die immobilisierbare Nukleinsäuresonde zwei oder mehr durch immobilisierbare Gruppen modifizierte Nukleotide enthält, die in der Nukleotidsequenz nicht direkt benachbart sind.

Weiterer Gegenstand ist die Verwendung der in dem Verfahren genannten Nukleinsäuren sowie ein System zum Nachweis von Nukleinsäuren.

Analytnukleinsäure im Sinne der Erfindung ist eine Nukleinsäure, die nachgewiesen werden soll. Sie befindet sich meist in einer Probe, welche noch weitere Bestandteile aufweist, beispielsweise einem Gewebe oder einer Flüssigkeit. Insbesondere befinden sich in der Probe noch weitere Nukleinsäuren, die nicht nachgewiesen werden sollen. Bei der Nukleinsäure kann es sich um jede Art von Nukleinsäure handeln, beispielsweise DNA, RNA oder Fragmente hiervon. Falls die Analytnukleinsäure ursprünglich in der Probe doppelsträngig vorliegt, wird sie bevorzugt in die Einzelstrangform überführt. Falls die Analytnukleinsäure an eine Festphase gebunden vorliegt, wird sie bevorzugt in Lösung gebracht.

Unter einer nachweisbar markierten Nukleinsäure wird eine Nukleinsäure verstanden, deren Anwesenheit oder Menge mit Hilfe eines Detektionssystems ermittelt werden kann. Dazu weist eine nachweisbar markierte Nukleinsäure gegenüber einer normalen Nukleotidsequenz Modifikationen auf. Geeignete Modifikationen sind dem Fachmann geläufig. Beispiel für eine Modifikation sind chemische Gruppen, die normalerweise in Nukleinsäuren nicht vorhanden sind, wie Enzyme, Farb- oder Fluoreszenzmoleküle, sowie Liganden, bevorzugt immunologisch bindefähige Reste. Zu den letztgenannten gehören insbesondere Haptene, wie Digoxigenin, aber auch Vitamine, wie Biotin. Bei den Enzymen wird der Nachweis meist über eine von dem Enzym katalysierte Farbbildungsreaktion geführt. Farbige oder fluoreszierende Moleküle können direkt photometrisch oder fluorometrisch nachgewiesen werden. Liganden werden meist mit einem entsprechenden enzym- oder farbmarkierten Rezeptor, der eine Affinität für den Liganden hat, in Kontakt gebracht und darüber nachgewiesen.

Unter einem nachweisbar markierten Nukleinsäurehybrid wird ein teilweise doppelsträngiges Hybrid von mindestens zwei Nukleinsäuren verstanden. Dabei können beide Nukleinsäuren, oder nur eine von ihnen, wie oben beschrieben, nachweisbar markiert sein. Wichtig ist, daß das Nukleinsäurehybrid einen einzelsträngigen Bereich aufweist. Bevorzugt besteht das Hybrid bei einem sogenannten Sandwich-Test aus der (nicht markierten) Analytnukleinsäure und einer nachweisbar markierten Detektorsonde.

Unter einer immobilisierbaren Nukleinsäuresonde wird eine Nukleinsäuresonde verstanden, welche durch Modifikation mit einem in üblichen Nukleinsäuren nicht vorhandenen Rest modifiziert sind. Solche Reste sind Reste, welche eine Affinität zu einem anderen Stoff besitzen. Beispiele sind Partner einer biospezifischen Reaktion zwischen einem Liganden und einem Rezeptor, beispielsweise einer immunologischen Reaktion, einer Reaktion zwischen Zucker und Lectin oder zwischen Vitamin und Bindeprotein. Besonders bevorzugte Reste sind Haptene oder Biotin. Im erfindungsgemäßen Verfahren ist der Rest an der immobilisierbaren Nukleinsäuresonde ein anderer als der nachweisbare Rest der nachweisbar markierten Nukleinsäure.

Die im folgenden beschriebenen Schritte des Nachweisverfahrens sind als Einzelschritte bekannt oder können analog zu bekannten Verfahren durchgeführt werden. Es wird insbesondere auf Molecular Cloning, Editor Sambrook et al., CSH 1989 Bezug genommen. Dazu gehören auch die bekannten Methoden zur Herstellung von markierten Nukleosidtriphosphaten, die chemische Synthese von modifizierten und unmodifizierten Oligonukleotiden, die Auswahl von Hybridisierungsbedingungen durch welche eine Spezifität erreicht werden kann, die vom Ausmaß der Homologie zwischen den zu hybridisierenden Nukleinsäuren deren GC-Gehalt und deren Länge abhängt, sowie die Bildung von Nukleinsäuren aus Nukleosidtriphosphaten mit Hilfe von Polymerasen ggf. unter Verwendung von sogenannten Primern. Das wesentliche an der Erfindung ist die Verwendung einer speziellen Fangprobe.

In einem ersten Schritt des erfindungsgemäßen Verfahrens wird unter Verwendung der Analytnukleinsäure eine nachweisbar markierte Nukleinsäure oder ein nachweisbar markiertes Nukleinsäurehybrid hergestellt. Dies kann beispielsweise geschehen, indem die Analytnukleinsäure selbst nachweisbar markiert wird. Dies kann geschehen durch den enzymatischen Einbau von markierten Nukleosidtriphosphaten oder die Verlängerung der Analytnukleinsäure mit markierten Nukleosidtriphosphaten (Tailing). Eine weitere Möglichkeit ist die Amplifikation eines Bereiches der Analytnukleinsäure unter Einbau nachweisbar markierter Mononukleosidtriphosphate oder nachweisbar markierter Oligonukleotide. Dazu sind aus dem Stand der Technik viele Methoden bekannt.

Eine Möglichkeit besteht in der Durchführung einer Amplifikation durch die Polymerase Chain Reaction gemäß EP-A-0 200 362 unter Verwendung der Analytnukleinsäure als Template-Nukleinsäure. Hier wird unter Verwendung zweier Primer, von denen einer komplementär zu einem Bereich der Analytnukleinsäure ist, und von denen der andere komplementär zu einem Teil des Gegenstrangs der Analytnukleinsäure ist, durch Verlängerung der Primer am Template eine Vielzahl von Kopien des Stranges und Gegenstranges der Analytnukleinsäure gebildet. Zur Herstellung der nachweisbar markierten Nukleinsäure wird mindestens ein nachweisbar markiertes Mononukleosidtriphosphat bei der Verlängerung der Primer oder ein markierter Primer eingesetzt.

Eine weitere Möglichkeit ergibt sich aus der Anwendung der Ligase Chain Reaction gemäß WO 90/06376, wenn mindestens eines der Oligonukleotide nachweisbar markiert ist.

Eine weitere Möglichkeit ist in der EP-A-0 329 822 beschrieben. Dabei wird unter Einbau eines promoterhaltigen Primers ein zu wesentlichen Teilen der Analytnukleinsäure komplementärer DNA-Strang gebildet. Nach Abbau der Analytnukleinsäure wird ein transkribierbarer Doppelstrang erzeugt, mit welchem unter Einbau markierter Mononukleosidtriphosphate eine Vielzahl von nachweisbar markierten Transkripten gebildet werden kann.

Als besonders effizient hat sich das erfindungsgemäße Verfahren in Form eines Sandwich-Nukleinsäure-Assays z.B. gemäß EP-A-0 192 168 erwiesen. Von den bekannten Sandwich-Assays unterscheidet sich das erfindungsgemäße Verfahren dadurch, daß eine besonders geartete Fangprobe verwendet wird.

In einem weiteren Schritt wird die nachweisbar markierte Nukleinsäure bzw. das nachweisbar markierte Nukleinsäurehybrid mit einer immobilisierbaren Nukleinsäuresonde unter Hybridisierungsbedingungen in Kontakt gebracht.

Die erfindungsemäß immobilisierbare Nukleinsäuresonde enthält zwei oder mehr durch immobilisierbare Gruppen modifizierte Nukleotide, die in der Nukleotidsequenz nicht direkt benachbart sind. Entscheidend ist neben der Anzahl der Liganden (Reportergruppen) der Abstand der Liganden zueinander, der bevorzugt 10 oder mehr Nukleotide betragen sollte, um diesen Effekt beobachten zu können. Beispielsweise ergaben Oligodesoxyribonukleotidsonden, die 5 Biotinreste (Biotin entspricht Ligand) direkt hintereinander gekoppelt enthielten und als Fangprobes zur Wandbindung eines Analyten an eine Streptavidinmatrix eingesetzt wurden, keinen Sensitivitätsgewinn im Gesamtassay gegenüber der nur mit einem Biotinrest verknüpften Vergleichsprobe (siehe Beispiel 2). Die "Fangprobe" kann ein Oligo- oder Polynukleotid (DNA oder RNA) sein, das in geeigneter Weise mit der nachzuweisenden Analytnukleinsäure hybridisieren kann. Sie kann einzelsträngig (z.B. Oligodesoxyribonukleotid, Oligoribonukleotid) oder doppelsträngig (z.B. Plasmid, Fragment) sein. Im letzteren Fall muß vor der Hybridisierung mit dem Analyten eine Denaturierung der Fangprobe erfolgen. Eine besonders bevorzugte Ausführungsform benutzt Nukleinsäuresonden, wobei jeweils die Endnukleotide der Nukleinsäuresonde modifziert sind.

Bevorzugt wird ein Oligodesoxynukleotid mit einer Länge zwischen 11 und 40 Nukleotiden, wobei die Markierung sinnvollerweise am 3'- und 5'-Ende angebracht ist, um den wie oben erwähnt, benötigten Abstand einhalten zu können.

Die immobilisierbaren Reste können prinzipiell sowohl am Basenteil oder am Zuckerteil oder am Phosphatteil der Nukleotide gebunden sein. Solche immobilisierbaren Nukleinsäuresonden können nach bekannten Methoden oder analog zu bekannten Methoden hergestellt werden. Die Anbringung eines Liganden an basenmodifizierte Nukleotide ist beispielsweise beschrieben in Nucleic Acids Res. 15 (12), S. 4857 bis 4876 (1987); Nucleic Acids Res. 16 (9), S. 4077 bis 4095 (1988); Nucleic Acids Res. 17, S. 4643 bis 7650 (1989) oder durch Einführung eines den Liganden bereits enthaltenden modifzierten Nukleotids (z.B. Nucleic Acids Res. 18 (15), S. 4355 bis 4360 (1990). Eine weitere geeignete chemische Methode um mehrere primäre Aminogruppen über die dann die Bindung an einen immobilisierbaren Rest erfolgen kann, in synthetische Oligonukleotide zu inkorporieren, ist in Nucleic Acids Res. 17 (18), S. 7179 bis 7194 (1989) beschrieben. Die Anbringung des Liganden an eine Nukleinsäure kann auch nach der Bisulfit-katalysierten Transaminierung (Nucleic Acids Res. 12 (2), S. 989 bis 1002 (1984) ) erfolgen. Die Anbringung mehrerer immobilisierbarer Reste kann auch nach enzymatischen Methoden erfolgen, z.B. durch Tailing unter Verwendung eines den entsprechenden Rest enthaltenden Nukleosidtriphosphats (DNA 5 (4), S. 333 bis 337 (1986) ) oder durch Random-Priming (Analyt. Biochem. 132, S. 6 1983).

Besonders bevorzugt im Sinne der Erfindung sind chemisch synthetisierte Oligonukleotide, da sie den Vorteil haben, daß der Abstand zwischen zwei modifizierten Nukleotiden genau vorherbestimmt und festgelegt werden kann.

Mehrfach markierte Nukleinsäuren waren bisher zur Verwendung als Detektionsprobe bekannt. Beispielsweise wird in EP-A-0 330 221 ein Oligo- oder Polynukleotid mit Biotin markiert und anschließend Biotin über einen Streptavidin-Enzym-Komplex nachgewiesen. Die Trennung zweier Biotin-dUMP-Reste in der Nukleotidsequenz durch gleichzeitigen Einbau von dTTP wird als eher ungeeignet beschrieben. In Nucleic Acids Res. 18 (15) S. 4358 ist gezeigt, daß durch Anbringen mehrerer immobilisierbarer Reste an Nukleinsäuren deren Bindung an feste Phasen verbessert werden kann.

Auch in Nucleic Acids Res. 18, S. 4345 bis 4354 (1990) werden mehrfach markierte Nukleinsäuren als Nachweisprobe verwendet. Die Herstellung mehrfach markierter Nukleinsäuren, bei denen die Markierung am Phosphatrest angebracht ist, ist in der WO 90/08838 beschrieben.

Wenn in der Probe Analytnukleinsäure enthalten ist, bildet sich aus der nachweisbar markierten Nukleinsäure oder dem nachweisbar markierten Nukleinsäurehybrid und der immobilisierbaren Nukleinsäuresonde ein Nukleinsäurehybrid. Dieses wird in dem erfindungsgemäßen Verfahren über die immobilisierbaren Reste an eine feste Phase gebunden. Die feste Phase enthält an ihrer Oberfläche Gruppen, welche eine Affinität zu dem immobilisierbaren Rest der Nukleinsäuresonde besitzen, beispielsweise einen Rezeptor. Falls die immobilisierbare Gruppe ein Hapten ist, enthält die feste Phase bevorzugt einen Antikörper gegen dieses Hapten. Im Falle von Biotin enthält die Festphase ein Biotinbindeprotein wie Avidin oder Streptavidin.

Die Menge an immobilisierbarem Hybrid ist nun ein Maß für die Menge oder die Anwesenheit der Analytnukleinsäure. Diese Menge kann über die Menge an Markierung, welche an die feste Phase immobilisiert ist, nachgewiesen werden. Dies geschieht bevorzugt auf bekannte Weise und richtet sich nach der Art der verwendeten Markierung. Vor der Durchführung der Nachweisreaktion wird bevorzugt die feste Phase von der flüssigen Phase entfernt. Gleichzeitig führt diese dazu, daß die Ausgangsstoffe der Herstellung der nachweisbar markierten Nukleinsäure oder des nachweisbar markierenden Nukleinsäurehybrids oder ein Überschuß an nachweisbar markierter Nukleinsäuresonde im Falle einer Sandwich-Hybridisierung mit der Flüssigkeit entfernt wird. Der Nachweis einer Nukleinsäure nach dem erfindungsgemäßen Verfahren, insbesondere wenn der Nachweis quantitativ geführt werden soll, umfaßt den Vergleich des Meßsignals, welches mit der Probe mit unbekanntem Analytnukleinsäuregehalt erhalten wurde, mit dem Meßsignal oder Meßsignalen, die mit einer oder mehreren Proben bekannten Analytnukleinsäuregehaltes erhalten wurden. Bevorzugt ist die Verwendung einer Eichkurve, welche auch in Form eingegebener Daten zur Verfügung stehen kann.

Ein weiterer Gegenstand der Erfindung ist die Verwendung einer Nukleinsäure, welcher zwei oder mehr durch immobilisierbare Gruppen modifizierte Nukleotide enthält, die in der Nukleotidsequenz nicht direkt benachbart sind, zur Immobilisierung von Nukleinsäuren. Dieser Gegenstand beruht auf der Tatsache, daß die erfindungsgemäßen Nukleinsäuresonden eine überraschenderweise bessere Immobilisierung gewährleisten. Das Verfahren kann beispielsweise bei der Affinitätstrennung von Nukleinsäuren eingesetzt werden.

Ein weiterer Gegenstand der Erfindung ist ein System zum Nachweis von Nukleinsäuren, welches ein oder mehrere Nukleinsäuren, die zwei oder mehr durch immobilisierbare Gruppen modifizierte Nukleotide enthalten, die in der jeweiligen Nukleotidsequenz nicht direkt benachbart sind, und mindestens eine Festphase, welche eine spezifische Affinität für die immobilisierbaren Gruppen der Nukleinsäure aufweist, enthält. Die Nukleinsäuren und die Festphase liegen in dem System vor dem Start eines Nachweisverfahrens bevorzugt getrennt voneinander vor. Außerdem kann das System noch weitere Bestandteile enthalten, die zum Nachweis von Nukleinsäuren erforderlich oder hilfreich sind. Dazu gehören insbesondere pH-Puffer und Reagenzien zum Nachweis der Markierung.

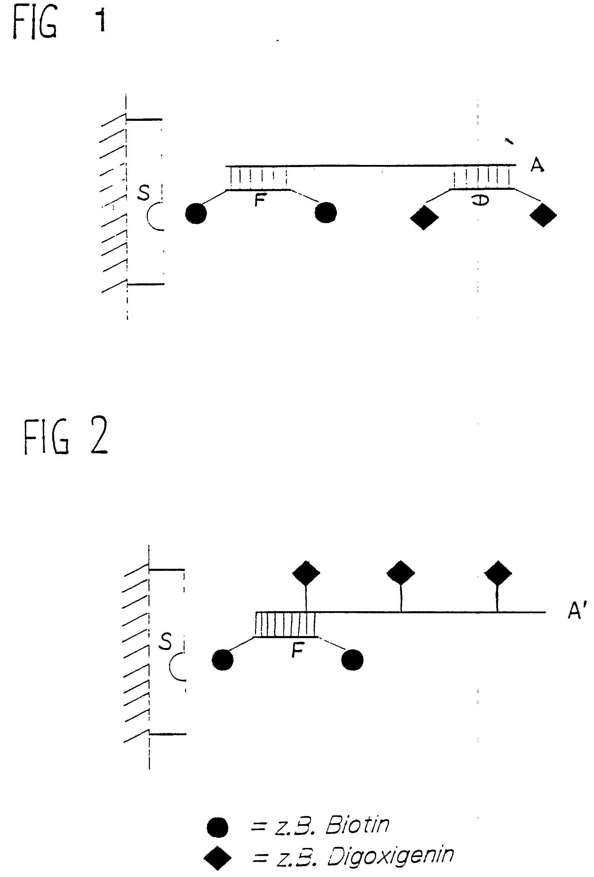

Fig. 1 zeigt schematisch die Bindung einer Analytnukleinsäure A über eine erfindungsgemäße Fangprobe F an eine mit Streptavidin S überzogene feste Phase in einem Sandwichtest. Die nachweisbar (mit einem Rautensymbol) markierte Detektorsonde ist mit D bezeichnet.

Fig. 2 zeigt die Wandbindung einer markierten Analytnukleinsäure A' über eine erfindungsgemäße Fangprobe an eine mit Streptavidin S überzogene feste Phase.

Fig. 3 verdeutlicht die Erhöhung der Empfindlichkeit eines Sandwich-Nukleinsäuretests, bei dem die immobilisierbaren Gruppen an benachbarten Nukleotiden befindlich sind, durch die erfindungsgemäße Trennung der Gruppen. Es ist deutlich, daß der Sensitivitätsgewinn bei Verwendung nur zweier Biotinreste jeweils an den Enden des Oligonukleotids sogar gegenüber der Verwendung von 10 Biotinresten an einem Ende eine unerwartete Sensitivitätssteigerung erbringt (siehe auch Beispiel 2).

Figur 4 zeigt die Sensitivitätssteigerung im Sandwichtest bei der Verwendung von zwei Biotinresten gegenüber der Verwendung von einem Biotinrest bei einer Oligonukleotidlänge von 30 Nukleotiden.

Figur 5 zeigt den Sensitivitätsvergleich von Figur 4, jedoch mit Oligonukleotiden mit einer Länge von 20 nt.

Figur 6 zeigt den Sensitivitätsvergleich für ein Verfahren, bei dem eine Analytnukleinsäure durch PCR amplifiziert und gleichzeitig markiert wurde, und bei der als Fangprobe Nukleinsäuresonden mit einer Länge von 15 Nukleotiden mit einem bzw. zwei Biotinresten verwendet wurden.

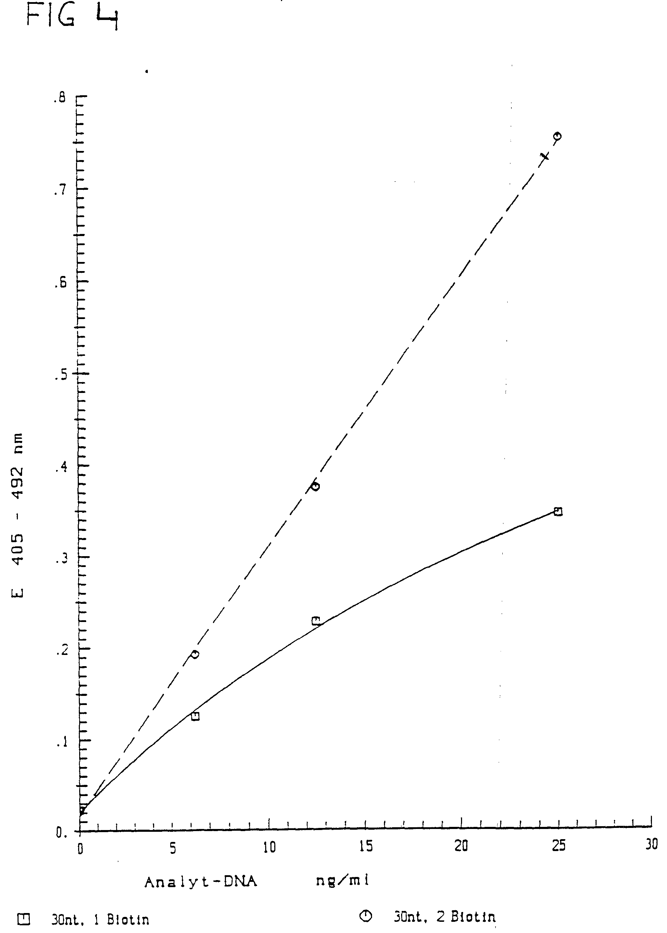

Die in Figur 4 bis 6 gezeigten Kurven für die erfindungsgemäßen Tests können gleichzeitig als Eichkurven für die Bestimmung einer unbekannten Menge einer Analytnukleinsäure verwendet werden.

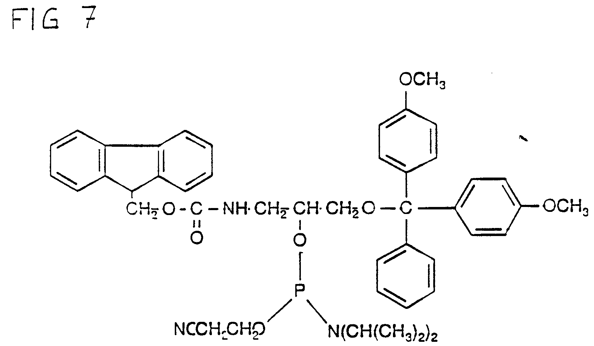

Fig. 7 zeigt die Formel von Aminomodifier II.

Die nachfolgend geschilderten Beispiele erläutern die Erfindung näher.

### Beispiel 1

### Herstellung von Oligonukleotiden

### A. Erläuterungen

Alle Oligonukleotide wurden mit Hilfe eines DNA-Synthesizers 8700 der Firma Biosearch nach der von Caruthers et al. Methods Enzymol. 154, 287, 1987 publizierten "Phosphoramiditmethode" hergestellt.

Die eingesetzten β-Cyanoethyl-Nucleosidphosphoramidite wurden von Roth (Karlsruhe, BRD) bezogen, D-Biotinoyl--aminocapronsäure-N-hydroxysuccinimidester von Boehringer Mannheim (Mannheim, BRD), Aminomodifier 2 (AM II) von der Firma Beckmann Instruments GmbH (Fullerton, CA, USA). Alle weiteren Reagentien und Lösungsmittel wurden in der besten Qualität die erhältlich ist, eingesetzt.

Die Reinigung der biotinylierten Oligonukleotide erfolgte mittels HPLC (Spectra Physics, Darmstadt, BRD) unter Verwendung einer Ionenaustauschsäule Mono Q HR 5/5 von Pharmacia (Freiburg, BRD) oder einer C₁₈-Säule (LiChrosorb RP 18-5, CS-Chromatographie Service, Langerwehe, BRD). Zur Dialyse wurden Spectra/Por 1000-Membranen (Roth, Karlsruhe, BRD) verwendet.

### B) Synthese und Reinigung der biotinylierten Oligonucleotide

Die biotinylierten Oligonukleotide wurden alle im 1 uMol Maßstab mit dem Trityl-off sowie Cleave-off Programm im Biosearch Synthesizer hergestellt. Da kein Trägermaterial mit Aminomodifier 2 zur Verfügung stand, wurde ein Thymidinträger verwendet und anschließend Aminomodifier 2 (siehe Fig. 7) nach dem Standardkupplungsprogramm (0.1 M in Acetonitril) ankondensiert.

Am 5'-Ende wurde nach dem Aminomodifier ein weiteres Thymidin gekoppelt, um zu verhindern, daß die primäre Hydroxylgruppe des Aminomodifiers während der Ammoniakbehandlung angegriffen und der Glycerylrest teilweise wieder abgespalten wird. Nach Beendigung der Synthese und Abspaltung der Schutzgruppen mit konz. NH₃-Lösung bei 55°C/5h wurden alle Lösungsmittel im Vaccum entfernt, der Rückstand in Wasser aufgenommen und das Produkt durch Ionenaustauschchromatographie (Mono Q HR 5/5; A: 0.25 mM Tris/HCl, 0.3 M NaCl; B: 0.25 Tris/HCl, 1 M NaCl; in 60 Min von 0 auf 100% B, Fluß 1 ml/Min) gereinigt. Anschließend wurde das Oligodesoxynukleotid durch Dialyse (Spectra/Por 1000, Roth) entsalzt. Die Biotinylierung der Oligonukleotide erfolgte anschließend, in dem 5-10 O.D._{260 nm} der Oligodesoxynukleotide in 0.5 ml 0.05 M K₂ HPO₄ /KH₂ PO₄-Puffer aufgenommen und mit einer Lösung aus 5 mg D-Biotinoyl- -aminocapronsäure-N-hydroxysuccinimidester in 0.5 ml DMF versetzt wurden. Der Reaktionsansatz wurde bei 37°C über Nacht inkubiert, anschließend die Lösungsmittel im Vaccum abgezogen, der Rückstand in bidest. Wasser aufgenommen und von überschüßigem Biotin abfiltriert. Die weitere Reinigung erfolgte mittels Reversed Phase HPLC mit einem Gradienten aus A: 0.1 M Triethylammoniumacetat pH 7; 5% Acetonitril, B: 0.1 M Triethylammoniumacetat pH 7; 40% Acetonitril. Bei einem Fluß von 2 ml/Min wurde innerhalb von 40 Min von 20% B auf 80% B gefahren. Der Unterschied in der Retentionszeit zwischen den doppelt biotinylierten Oligonukleotiden und den entsprechenden Aminomodifiermodifizierten Ausgangsoligodesoxynukleotiden betrug in Abhängigkeit von der Sequenzlänge zwischen 2 und 4 Minuten. Nach erneuter Dialyse konnten die biotinylierten Oligodesoxynukleotide in 1.5 bis 3.5 O.D.₂₆₀ erhalten werden.

### Beispiel 2

### Nachweis von HBV-Analyt-DNA im Sandwich-Format unter Berücksichtigung des Modifizierungsortes

Der Test erfolgt in einem Sandwich unter Bindung der Analyt-DNA an eine Festphase. Als Analyt-DNA wird eine Teilsequenz der Hepatitis B-Virus DNA verwendet, die in einem Plasmid kloniert vorliegt. Durch ein Biotinmarkiertes Oligonukleotid, dessen Sequenz komplementär zu einem Bereich der HBV-DNA ist, wird die Analyt-DNA an eine Streptavidin-Festphase gebunden. Der Nachweis der gebundenen Analyt-DNA erfolgt dann über ein Digoxigenin markiertes Oligonukleotid, das ebenfalls komplementär zu einem anderen Bereich der Analyt-DNA ist. Dieses kann durch anti-Digoxigenin-Antikörper, die mit Meerrettichperoxidase konjugiert sind, erkannt werden und das Hybrid anschließend durch eine enzymkatalysierte Farbbildungsreaktion nachgewiesen werden.

Es wird eine Verdünnungsreihe des Plasmids in einem Bereich von 4,4 ug/ml, 2,2 ug/ml, 1,1 ug/ml, 0,55 ug/ml in TE-Puffer (10 mM Tris-HCl, 1 mM EDTA, pH 7,5) hergestellt. Zur Denaturierung der Doppelstrang-DNA werden 90 ul der Plasmid-Lösung mit 10 ul 5 M NaOH versetzt und 10 min bei Raumtemperatur inkubiert. 20 ul des Denaturierungsansatzes werden anschließend in eine Kavität einer mit Streptavidin beschichteten Mikrotiterplatte pipettiert und sofort durch Zugabe von 180 ul Hybridisierungslösung (50 mM Na-Phosphatpuffer, 0,75 M NaCl, 0,075 M Na-Citrat, 0,05% Rinderserumalbumin, pH 5,4) neutralisiert. Daraus ergeben sich folgende Konzentrationen des Plasmids im Test: 50, 100, 200, 400 ng/ml. Die Hybridisierungslösung enthält zusätzlich jeweils 200 ng/ml eines Digoxigenin-markierten Oligonukleotides (1fach mit Digoxigenin am 5'-Ende markiert, Position 287c-248c im HBV-Genom) und eines Biotin-markierten Oligonukleotides (Position 2456c-2417c im HBV-Genom). Der Test wird mit Oligonukleotiden mit verschiedenen Biotin-Markierungsgraden durchgeführt:

HBV-Oli 7-4 (1 Biotin, 40mer), SEQ ID NO 1:
5′-T-(Bio-AMII)-CATTGAGATTCCCGAGATTGAGATCTTCTGCGACGCGGCG-
3′

HBV-Oli 7-5 (5 Biotin, 40mer), SEQ ID NO 2:
5'-T-(Bio-AMII)5-CATTGAGATTCCCGAGATTCAGATCTTCTGCGACGCGGCG-
3'

HBV-Oli 7-6 (10 Biotin, 40mer), SEQ ID NO 3:
5'-T-(Bio-AMII)10-
CATTGAGATTCCCGAGATTGAGATCTTCTGCGACGCGGCG-3'

HBV-Oli 7-7 (2 Biotin, 40 mer), SEQ ID NO 4:
5'-T-(Bio-AMII)-CATTGAGATTCCCGAGATTCAGATCTTCTGCGACGCGGCG-
(AMII-Bio)-T-3'

Der Hybridisierungsansatz wird 3 h bei 37°C in der Mikrotiterplatte unter Schütteln inkubiert. Nach Absaugen der Lösung wird 2 x 10 min mit 0,3 M NaCl, 0,03 M Na-Citrat, 0,2 % Na-Dodecylsulfat bei 37°C und anschließend 1 x kurz bei Raumtemperatur mit 0,9 % NaCl gewaschen, um nicht gebundene Reaktionspartner aus dem Test zu entfernen. 20 mU/ml eines anti-Digoxigenin-Antikörper-Meerrettichperoxidase-Konjugates in 100 mM Tris-HCl (pH 7,5), 0,9 % NaCl, 1 % Rinderserumalbumin werden zugegeben und 30 min bei 37°C unter Schütteln inkubiert. Nicht gebundenes Konjugat wird durch 3maliges kurzes Waschen mit 0,9 % Nacl bei Raumtemperatur entfernt. Durch Zugabe der Substratlösung ABTS^{E} (1,9 mM, 2,2'Azino-di-[3-ethylbenzthiazolin-sulfonsäure (6)]-diammoniumsalz) wird die Nachweisreaktion gestartet. Die Inkubation erfolgt für 30 min bei 37°C unter Schütteln. Die Extinktion wird anschließend mittels eines ELISA-Readers bei 405 nm gemessen.

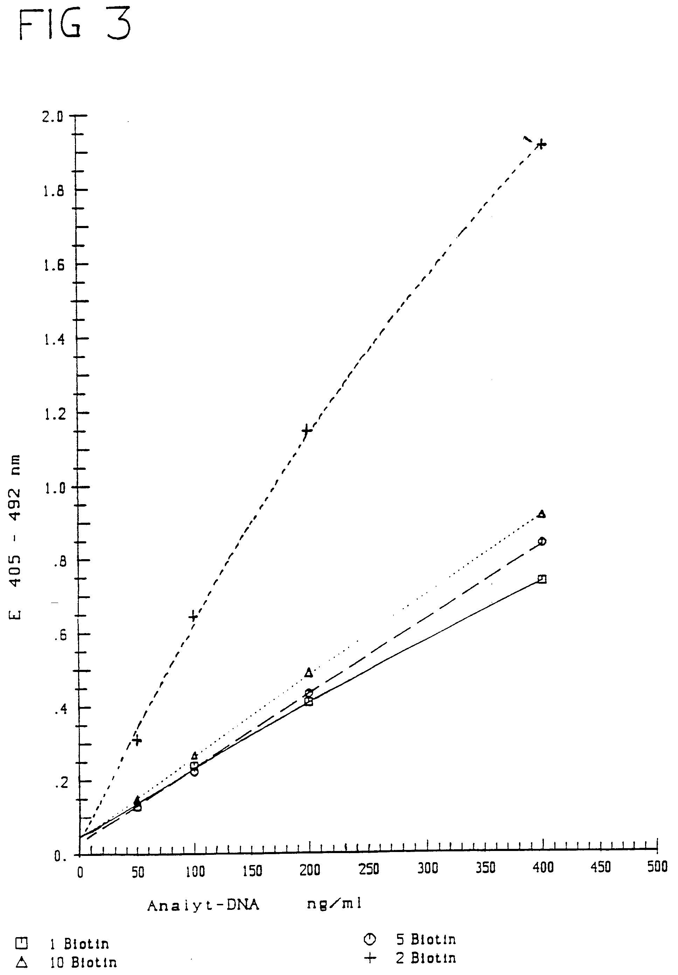

Die Ergebnisse sind in Fig. 3 dargestellt.

### Beispiel 3

### Nachweis von HBV-Analyt-DNA im Sandwich-Format unter Berücksichtigung der Kettenlänge und des Modifizierungsortes

Es wird eine Verdünnungsreihe des Plasmids, das HBV-spezifische Sequenzen enthält, im Bereich von 62,5 bis 250 ng/ml in 10 mM Tric-HCl, 1 mM EDTA, pH 7,5 hergestellt. 90 ul der Plasmidlösung werden mit 10 ul 5 M NaOH versetzt und 10 min bei Raumtemperatur inkubiert. 20 ul dieses Denaturierungsansatzes werden in eine mit Streptavidin beschichtete Kavität einer Mikrotiterplatte pipettiert und mit 180 ul Hybridisierungslösung (50 mM Phosphatpuffer, 0,75 M NaCl, 0,075 M Na-Citrat, 0,05% Rinderserumalbumin (RSA), pH 5,4) versetzt. Der Hybridisierungslösung sind jeweils 200 ng/ml des Biotin-markierten Fang-Oligonukleotides und des Digoxigenin-markierten Oligonukleotides (Pos. 405-444 im HBV-Genom, 40mer, 2fach mit Digoxigenin am 3'und 5' Ende markiert) zugesetzt. Die Analyt-DNA-Konzentration im Test beträgt somit 6,25, 12,5, 25 und 50 ng/ml im Test.

HBV-Oli 25-3 (Pos. 2327-2356, 30mer, 1 Biotin),
SEQ ID NO 5:
5'-T-(Bio-AMII)-CACTTCCGGAAACTACTGTTGTTAGACGAC-3'

HBV-Oli 25-4 (Pos. 2327-2356, 30mer, 2 Biotin),
SEQ ID NO 6:
5'-T-(Bio-AMII)-CACTTCCGGAAACTACTGTTGTTAGACGAC-(AMII-Bio-
)-T-3'

HBV-Oli 33-1 (Pos. 2332-2351, 20mer, 1 Biotin).
SEQ ID NO 7:
5'-T-(Bio-AMII)-CCGGAAACTACTGTTGTTAG-3'

HBV-Oli 33-2 (Pos. 2332-2351, 20mer, 2 Biotin),
SEQ ID NO 8:
5'-T-(Bio-AMII)-CCGGAAACTACTGTTGTTAG-(AMII-Bio)-T-3'

Die angegebenen Positionen beziehen sich auf das HBV-Genom.

Der Hybridisierungsansatz wird 3 h bei 37°C geschüttelt. Anschließend wird 2 x 10 min mit 0,3 M NaCl, 0,03 M Na-Citrat, 0,2% Na-Dodecylsulfat bei 37°C und dann 1 x kurz mit 0,9 %iger NaCl bei Raumtemperatur gewaschen. Anschließend werden 200 /ul des anti-Digoxigenin-Antikörper-Meerrettichperoxidase-Konjugates (200 mU/ml in 100 mM Tris-HCl, pH 7,5, 0,9 % NaCl, 1 % RSA) zugegeben und 30 min unter Schütteln bei 37°C inkubiert. Nach dreimaligem Waschen mit 0,9 % Nacl wird die Substratlösung (1,9 mM ABTS^{E}) zupipettiert, wiederum 30 min bei 37°C geschüttelt und dann die Extinktion bei 405 nm mittels eines ELISA-Readers gemessen.

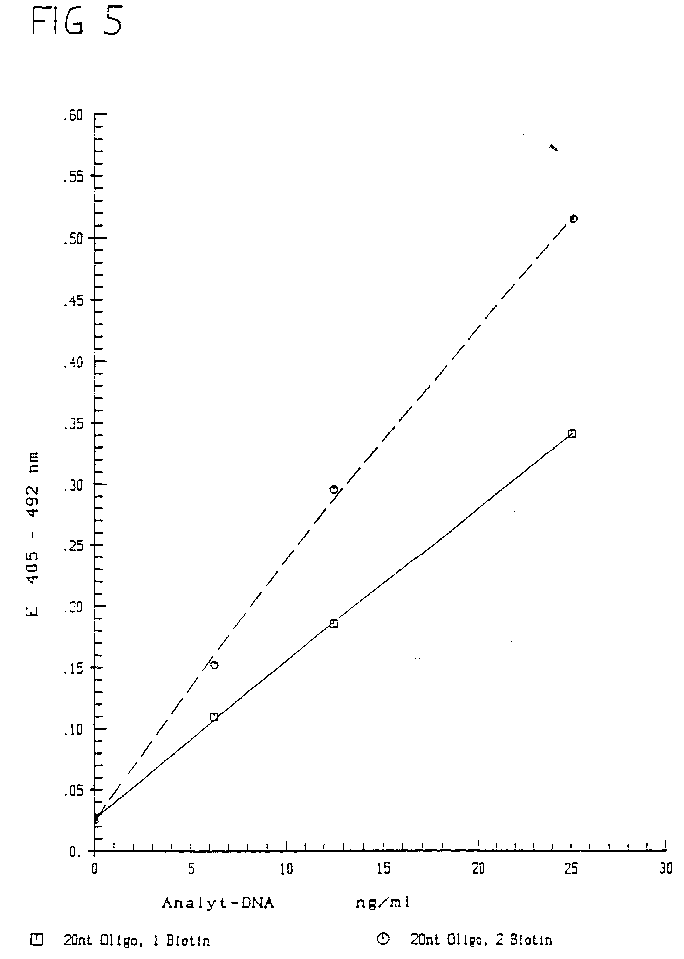

Die Ergebnisse für die 30 nt Oligonukleotide sind in Fig. 4, die für die 20 nt Oligonukleotide in Fig. 5 dargestellt.

### Beispiel 4

### Nachweis von PCR-Produkten mit ein- und zweifach markierten Oligonukleotiden

Zum DNA-Nachweis der Hepatitis B-Viren wird zunächst die virale DNA mittels der Polymerase Chain Reaction (EP-A-0 200 362) vermehrt. Während dieser Amplifikation wird Digoxigenin in Form von Dig-11-dUTP (EP-A-324474) in die DNA eingebaut. Dieses Hapten erlaubt anschließend in einem heterogenen Immunoassay einen empfindlichen Nachweis des PCR-Produktes. Die Wandbindung der Digoxigenin-markierten DNA an die mit Streptavidin beschichteten Röhrchen erfolgt mittels eines Biotin-marierten Oligonukleotides, dessen Bereich komplementär zu einem Bereich des PCR-Produktes ist. Der Nachweis erfolgt wiederum mit einem anti-Digoxigenin-Antikörper-Meerrettichperoxidase-Konjugat und einer anschließenden Farbreaktion.

HBe-positives Humanplasma mit einem Virustiter von 1 x 10¹⁰ Hepatitis B-Viren pro ml wird in Normalserum verdünnt, so daß der Virusgehalt in den Verdünnungen bei 1 x 10⁷ Viren/ml liegt. Zur Lyse werden 10 µl der Virusverdünnung mit 10 µl 0,2 M NaOH versetzt und 1 h bei 37°C inkubiert. Der Lyseansatz wird durch Zugabe von 30 µl Neutralisationsmix (100 mM KCl, 50 mM Tris-HCl, pH 6,5, 3mM MgC12) neutralisiert. 20 /ul dieser Lösung wird in die anschließende Amplifikationsreaktion eingesetzt (Amplifikationsbedingungen: je 200 nM der PCR-Primer, je 200 µM dATP, dCTP, dGTP, 175 µM dTTP, 25 µM Digoxigenin-11-2'-dUTP, 2,5 U Thermus aguaticus DNA-Polymerase in 50 mM KCl, 10 mM Tris-HCl, pH 8,9, 1,5 mM MgCl₂ 0,01 % Gelatine; Gesamtvolumen 100 µl). Der Ansatz wird mit 100 µl Mineralöl überschichtet und in einem Thermo-Cycler (Perkin-Elmer) 30 Zyklen inkubiert:
30 sec 92°C, 30 sec 50°C, 60 sec 70°C.

Aufgrund der Lage der PCR-Primer (PCR-Primer 1: Position 1937-1960; PCR-Primer 2: Position 2434c-2460c im HBV-Genom) wird ein 500 bp großes DNA-Fragment erzeugt, das anschließend in einem Zweikomponenten-Testsystem in Streptavidin-beschichteten Mikrotiterplatten nachgewiesen wird.

Der PCR-Ansatz wird derart in H₂O verdünnt, daß bezogen auf die ursprüngliche Viruskonzentration von 1 x 10⁷ Viren pro mol Serum, Verdünnungen entstehen, die in einem Bereich von 1 x 10⁵ bis 1 x 10⁴ Viren/ml entsprechen. 20 µl des verdünnten PCR-Ansatzes wird in einer Endkonzentration von 0,1 N NaOH in einem Volumen von 50 µl 10 min bei Raumtemperatur zur Denaturierung inkubiert. 20 µl der Denaturierungslösung werden zusammen mit 180 µl Hybridisierungslösung (1 M NaCl, 0,1 M Na-Citrat, 67,5 mM Na-Phosphat, 0,05 % RSA, pH 6,7) in eine mit Streptavidin beschichtete Kavität einer Mikrotiterplatte pipettiert. Dem Hybridisierungspuffer sind die Biotin-markierten Oligonukleotide in einer Konzentration von 100 ng/ml zugesetzt:

HBV-Oli 34-1 (Pos. 2299-2313, 15mer, 1 Biotin),
SEQ ID NO 9:
5'-T-(AMII-Bio)-AGACCACCAAATGCC-3'

HBV-Oli 34-2 (Pos. 2299-2313, 15mer, 2 Biotin),
SEQ ID NO 10:
5'-T-(AMII-Bio)-AGACCACCAAATGCC-(Bio-AMIIH)-T-3'

Der Hybridisierungsansatz wird für 3 h bei 37°C unter Schütteln inkubiert. Anschließend wird die Lösung abgesaugt und die Kavität 3mal mit 0,9 % NaCl gewaschen. 200 µl einer Lösung von 200 mU/ml anti-Digoxigenin-Antikörper-Meerrettichperoxidase-Konjugat in 100 mM Tris-HC1, pH 7,5, 0,9 % NaCl, 1 % RSA werden zugegeben und 30 min bei 37°C inkubiert. Nach nochmaligem Waschen mit 0,9 % NaCl wird die Substratreaktion durch Zugabe von 200 µl ABTS^{R} gestartet. Die photometrische Messung erfolgt nach 30 Min bei 405 nm.

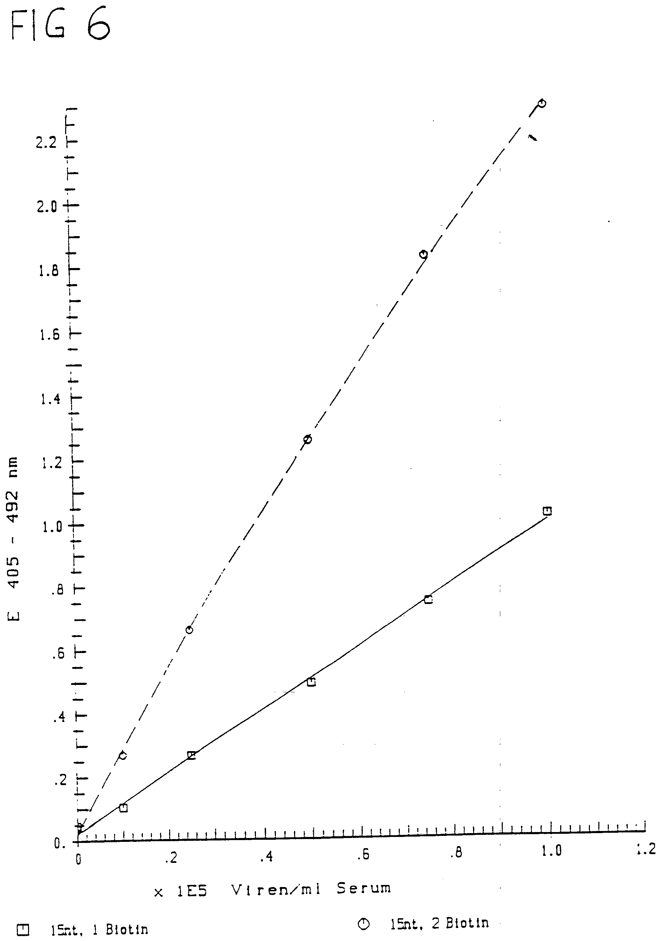

Die Ergebnisse sind in Fig. 6 dargestellt.

Sequenzprotokoll

SEQ ID NO 1

Sequenzlänge: 41 Basen
Art der Sequenz: Nukleotidsequenz
Strangform: Einzelstrang
Topologie: Linear
Art des Moleküls: Teil aus Genom-DNA mit
Modifikationen N
Position: HBV-Genom 2456c-2417c (Hpbadw-
Datenbank)
Antisense
Modifikation N: T daran über 3'-O Biotin mit
Aminomodifier II gebunden

5'-N CAT TGA GAT TCC CGA GAT TGA GAT CTT CTG CGA CGC GGC
G-3'

SEQ ID NO 2

Sequenzlänge: 41 Basen
Art der Sequenz: Nukleotidsequenz
Strangform: Einzelstrang
Topologie: Linear
Art des Moleküls: Teil aus Genom-DNA mit
Modifikationen N

Position:                    HBV-Genom 2456c-2417c
Antisense
Modifikation N:              T daran über 3'-O 5 Biotin mit
                             5 Aminomodifier II gebunden

5'-N CAT TGA GAT TCC CGA GAT TGA GAT CTT CTG CGA CGC GGC
G-3'

SEQ ID NO 3

Sequenzlänge:                41 Basen
Art der Sequenz:             Nukleotidsequenz
Strangform:                  Einzelstrang
Topologie:                   Linear
Art des Moleküls:            Teil aus Genom-DNA mit
                             Modifikationen N
Position:                    HBV-Genom 2456c-2417c
Antisense
Modifikation N:              T daran über 3'-O 10 Biotin mit
                             10 Aminomodifier II gebunden

5'-N CAT TGA GAT TCC CGA GAT TGA GAT CTT CTG CGA CGC GGC
G-3'

SEQ ID NO 4

Sequenzlänge:                42 Basen
Art der Sequenz:             Nukleotidsequenz
Strangform:                  Einzelstrang
Topologie:                   Linear

Art des Moleküls:                Teil aus Genom-DNA mit
                                  Modifikationen N
Position:                         HBV-Genom 2456c-2417c
Antisense
Modifikation N am                 T daran über 3'-O Biotin mit
5'-Ende:                          Aminomodifier II gebunden
Modifiaktion N am                 T daran über 5'-O Biotin mit
3'-Ende:                          Aminomodifier II gebunden

5'-N CAT TGA GAT TCC CGA GAT TGA GAT CTT CTG CGA CGC GGC
N-3'

SEQ ID NO 5

Sequenzlänge:                     31 Basen
Art der Sequenz:                  Nukleotidsequenz
Strangform:                       Einzelstrang
Topologie:                        Linear
Art des Moleküls:                 Teil aus Genom-DNA mit
                                  Modifikationen N
Position:                         HBV-Genom 2327-2356
Modifikation N:                   T daran über 3'-O Biotin mit
                                  Aminomodifier II gebunden

5'-N CAC TTC CGG AAA CTA CTG TTG TTA GAC GAC 3'

SEQ ID NO 6

Sequenzlänge: 32 Basen
Art der Sequenz: Nukleotidsequenz
Strangform: Einzelstrang
Topologie: Linear
Art des Moleküls: Teil aus Genom-DNA mit
Modifikationen N
Position: HBV-Genom 2327-2356
Modifikation N am T daran über 3'-O Biotin mit
5'-Ende: Aminomodifier II gebunden
Modifiaktion N am T daran über 5'-O Biotin mit
3'-Ende: Aminomodifier II gebunden

5'-N CAC TTC CGG AAA CTA CTG TTG TTA GAC GAC N-3'

SEQ ID NO 7

Sequenzlänge: 20 Basen
Art der Sequenz: Nukleotidsequenz
Strangform: Einzelstrang
Topologie: Linear
Art des Moleküls: Teil aus Genom-DNA mit
Modifikationen N
Position: HBV-Genom 2332-2351
Modifikation N: T daran über 3'-O Biotin mit
Aminomodifier II gebunden

5'-NC CGG AAA CTA CTG TTG TTA G-3'

SEQ ID NO 8

Sequenzlänge: 21 Basen
Art der Sequenz: Nukleotidsequenz
Strangform: Einzelstrang
Topologie: Linear
Art des Moleküls: Teil aus Genom-DNA mit
Modifikationen N
Position: HBV-Genom 2332-2351
Modifikation N am T daran über 3'-O Biotin mit
5'-Ende: Aminomodifier II gebunden
Modifiaktion N am T daran über 5'-O Biotin mit
3'-Ende: Aminomodifier II gebunden

5'-NC CGG AAA CTA CTG TTG TTA GN-3'

SEQ ID NO 9

Sequenzlänge: 16 Basen
Art der Sequenz: Nukleotidsequenz
Strangform: Einzelstrang
Topologie: Linear
Art des Moleküls: Teil aus Genom-DNA mit
Modifikationen N
Position: HBV-Genom 2299-2313
Modifikation N: T daran über 3'-O Biotin mit
Aminomodifier II gebunden

5'-N AGA CCA CCA AAT GCC-3'

SEQ ID NO 10

Sequenzlänge: 17 Basen
Art der Sequenz: Nukleotidsequenz
Strangform: Einzelstrang
Topologie: Linear
Art des Moleküls: Teil aus Genom-DNA mit
Modifikationen N
Position: HBV-Genom 2299-2313
Modifikation N am T daran über 3'-O Biotin mit
5'-Ende: Aminomodifier II gebunden
Modifiaktion N am T daran über 5'-O Biotin mit
3'-Ende: Aminomodifier II gebunden

5'-N AGA CCA CCA AAT GCC N-3'

## Patentansprüche

1. Verfahren zum Nachweis einer Analytnukleinsäure welches folgende Schritte enthält:
- Hybridisierung einer nachweisbar markierten Nukleinsäure oder eines nachweisbar markierten Nukleinsäurehybrids mit einer immobilisierbaren Nukleinsäuresonde;
- Immobilisierung des gebildeten Hybrids und
- Nachweis der Menge an immobilisiertem Hybrid über die Menge an Markierung
dadurch gekennzeichnet, daß die immobilisierbare Nukleinsäuresonde zwei oder mehr durch immobilisierbare Gruppen modifizierte Nukleotide enthält, die in der Nukleotidsequenz nicht direkt benachbart sind.

2. Verfahren gemäß Anspruch 1, dadurch gekennzeichnet, daß der Abstand zwischen den modifizierten Nukleotiden mindestens 10 Nukleotide beträgt.

3. Verfahren gemäß Anspruch 1, dadurch gekennzeichnet, daß der Abstand zwischen den modifizierten Nukleotiden zwischen 11 und 40 Nukleotide beträgt.

4. Verfahren nach einem der vorangegangenen Ansprüche, dadurch gekennzeichnet, daß die modifizierten Nukleotide jeweils die Endnukleotide der Nukleinsäuresonde darstellen.

5. Verfahren nach einem der vorangehenden Ansprüche, dadurch gekennzeichnet, daß die immobilisierbaren Reste an Phosphatreste der Nukleotide gebunden sind.

6. Verfahren nach einem der Ansprüche 1-4, dadurch gekennzeichnet, daß die immobilisierbaren Gruppen an den Basenteil der Nukleotide gebunden sind.

7. Verfahren nach einem der vorangehenden Ansprüche, dadurch gekennzeichnet, daß die nachweisbar markierte Nukleinsäure ein Amplifikat eines Bereiches der Analytnukleinsäure ist.

8. Verfahren nach einem der Ansprüche 1-6, dadurch gekennzeichnet, daß das nachweisbar markierte Hybrid ein Hybrid aus der Analytnukleinsäure und einer nachweisbar markierten Nukleinsäuresonde ist.

9. Verwendung einer Nukleinsäure, welche zwei oder mehr durch immobilisierbare Gruppen modifizierte Nukleotide enthält, die in der Nukleotidsequenz nicht direkt benachbart sind, zur Immobilisierung von Nukleinsäuren.

10. System zum Nachweis von Nukleinsäuren enthaltend
- eine oder mehrere Nukleinsäuren, die zwei oder mehr durch immobilisierbare Gruppen modifizierte Nukleotide enthalten, die in der jeweiligen Nukleotidsequenz nicht direkt benachbart sind und
- mindestens eine Festphase, welche eine spezifische Affinität für die immobilisierbaren Gruppen der Nukleinsäure aufweist.

## Claims

1. Process for the detection of an analyte nucleic acid which contains the following steps:
- hybridisation of a detectably labelled nucleic acid or of a detectably labelled nucleic acid hybrid with an immobilisable nucleic acid probe;
- immobilisation of the hybrid formed and
- detection of the amount of immobilised hybrid via the amount of the labelling,
characterised in that the immobilisable nucleic acid probe contains two or more nucleotides modified by immobilisable groups which are not directly neighbouring in the nucleotide sequence.

2. Process according to claim 1, characterised in that the distance between the modified nucleotides amounts to at least 10 nucleotides.

3. Process according to claim 1, characterised in that the distance between the modified nucleotides amounts to between 11 and 40 nucleotides.

4. Process according to one of the preceding claims, characterised in that the modified nucleotides each represent the end nucleotides of the nucleic acid probe.

5. Process according to one of the preceding claims, characterised in that the immobilisable groups are bound to phosphate residues of the nucleotides.

6. Process according to one of claims 1 - 4, characterised in that the immobilisable groups are bound to the base part of the nucleotides.

7. Process according to one of the preceding claims, characterised in that the detectably labelled nucleic acid is an amplification product of a region of the analyte nucleic acid.

8. Process according to one of claims 1 - 6, characterised in that the detectably labelled hybrid is a hybrid of the analyte nucleic acid and of a detectably labelled nucleic acid probe.

9. Use of a nucleic acid which contains two or more nucleotides modified by immobilisable groups which are not directly neighbouring in the nucleotide sequence for the immobilisation of nucleic acids.

10. System for the detection of nucleic acids containing
- one or more nucleic acids which contain two or more nucleotides modified by immobilisable groups which are not directly neighbouring in the particular nucleotide sequence and
- at least one solid phase which displays a specific affinity for the immobilisable groups of the nucleic acid.

## Revendications

1. Procédé de détection d'un acide nucléique analyte, comprenant les étapes consistant à :
- hybrider un acide nucléique marqué détectable, ou un hybride d'acide nucléique marqué détectable, à une sonde nucléique immobilisable ;
- immobiliser l'hybride formé et
- détecter la quantité d'hybride immobilisé au moyen de la quantité de marqueur,
caractérisé par le fait que la sonde nucléique immobilisable contient deux ou plus de deux nucléotides modifiés par des groupes immobilisables, qui ne sont pas immédiatement contigüs dans la séquence nucléotidique.

2. Procédé selon la revendication 1, caractérisé par le fait que la distance entre les nucléotides modifiés est d'au moins 10 nucléotides.

3. Procédé selon la revendication 1, caractérisé par le fait que la distance entre les nucléotides modifiés est comprise entre 11 et 40 nucléotides.

4. Procédé selon l'une quelconque des revendications précédentes, caractérisé par le fait que les nucléotides modifiés représentent chacun le nucléotide terminal de la sonde nucléique.

5. Procédé selon l'une quelconque des revendications précédentes, caractérisé par le fait que les restes immobilisables sont fixés sur des restes phosphate du nucléotide.

6. Procédé selon l'une quelconque des revendications 1-4, caractérisé par le fait que les groupes immobilisables sont fixés sur la partie basique du nucléotide.

7. Procédé selon l'une quelconque des revendications précédentes, caractérisé par le fait que l'acide nucléique marqué détectable est une amplification d'un domaine de l'acide nucléique analyte.

8. Procédé selon l'une quelconque des revendications 1-6, caractérisé par le fait que l'hybride marqué détectable est un hybride de l'acide nucléique analyte et d'une sonde nucléique marquée détectable.

9. Utilisation d'un acide nucléique, qui contient deux ou plus de deux nucléotides modifiés par des groupes immobilisables, qui ne sont pas immédiatement contigüs dans la séquence nucléotidique, pour immobiliser des acides nucléiques.

10. Système pour la détection d'acides nucléiques, comprenant
- un ou plusieurs acides nucléiques, qui contiennent deux ou plus de deux nucléotides modifiés par des groupes immobilisables, qui ne sont pas immédiatement contigüs dans la séquence nucléotidique concernée, et
- au moins une phase solide, qui présente une affinité spécifique pour les groupes immobilisables de l'acide nucléique.
